(19) Europäisches Patentamt
European Patent Office
Office européen des brevets

(11) **EP 3 755 291 B1**

(12) **FASCICULE DE BREVET EUROPEEN**

(45) Date de publication et mention de la délivrance du brevet:
**05.01.2022 Bulletin 2022/01**

(21) Numéro de dépôt: **19711974.6**

(22) Date de dépôt: **20.02.2019**

(51) Int Cl.:
*C09J 153/02* (2006.01)   *C08L 53/02* (2006.01)
*A61L 15/24* (2006.01)   *A61L 15/42* (2006.01)
*A61F 13/02* (2006.01)

(86) Numéro de dépôt international:
**PCT/FR2019/050385**

(87) Numéro de publication internationale:
**WO 2019/162612 (29.08.2019 Gazette 2019/35)**

(54) **COMPOSITION PRESENTANT UNE EXCELLENTE PERMEABILITE A LA VAPEUR D'EAU**

ZUSAMMENSETZUNG MIT AUSGEZEICHNETER WASSERDAMPFDURCHLÄSSIGKEIT

COMPOSITION HAVING EXCELLENT WATER VAPOR PERMEABILITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.02.2018 FR 1851442**

(43) Date de publication de la demande:
**30.12.2020 Bulletin 2020/53**

(73) Titulaire: **URGO RECHERCHE INNOVATION ET DEVELOPPEMENT
21300 Chenôve (FR)**

(72) Inventeurs:
• **DANÉROL, Anne-Sophie
21000 DIJON (FR)**
• **GUILLAMAUD, Christelle
21300 CHENOVE (FR)**
• **PERNOT, Jean-Marc
21000 DIJON (FR)**

(74) Mandataire: **Plasseraud IP
66, rue de la Chaussée d'Antin
75440 Paris Cedex 09 (FR)**

(56) Documents cités:
WO-A1-2011/135256   US-A- 6 080 797
US-A1- 2003 134 552   US-B1- 6 270 794

**Description**

**RESUME**

La présente invention est relative à une nouvelle composition comprenant au moins un copolymère à blocs styrèniques, au moins un plastifiant, et des particules d'un polymère réticulé ayant une densité de groupement carboxylate comprise entre 2,0 et 12,0 meq/g et une taille de pore moyenne comprise entre 0,005 et 1,0 $\mu$m, utilisable notamment pour la réalisation d'une matrice élastomérique convenant à tout dispositif, par exemple à visée médicale tel qu'un patch, un film, une bande ou un pansement, de préférence un pansement, ou bien encore convenant à tout dispositif de type textile fonctionnel tel qu'un article de sport.

La présente invention a trait tout particulièrement à une composition présentant une excellente perméabilité à la vapeur d'eau. Cette composition est notamment capable de mettre en œuvre cette excellente propriété dans des domaines techniques aussi variés que le domaine médical, les emballages agro-alimentaires, l'automobile, les articles techniques de sport ou de loisir ou encore le domaine du textile.

**ETAT DE LA TECHNIQUE ANTERIEURE**

Un pansement est assimilable notamment à un dispositif de protection permettant de recouvrir une plaie. Un pansement peut avoir plusieurs autres fonctions cumulables entre elles ou non, telles que:

- protéger la plaie et l'isoler du milieu extérieur ;
- permettre une meilleure cicatrisation en maintenant un milieu humide favorable sur le lit de la plaie ;
- faire cesser un saignement minime ;
- rapprocher les berges d'une plaie ;
- absorber et gérer les exsudats afin de préserver les berges de la plaie et la peau péri-lésionnelle.

La cicatrisation naturelle d'une plaie se déroule en trois phases successives, chacune de ces phases étant caractérisée par des activités cellulaires spécifiques et différentes : la phase de détersion, la phase de bourgeonnement (ou granulation) et la phase d'épithélialisation. Tout au long du processus de cicatrisation, la plaie produit des exsudats fluides ou visqueux qui doivent si possible être absorbés par un pansement ou évacués par ce dernier pour être guidés vers un réservoir à l'extérieur de la plaie (cas de la thérapie par pression négative).

Suivant la gravité de la plaie, le processus de cicatrisation peut s'étendre de quelques jours à plusieurs mois. Dans le cas de plaies particulièrement exsudatives, les exsudats fluides ou visqueux peuvent inonder le lit de la plaie et constituer un environnement favorable à la dégradation des tissus sains périlésionnels, du fait de la macération des tissus ou de leur surinfection.

Pour ce type de plaies, le rôle d'un pansement est donc d'absorber ou de gérer ces exsudats fluides pour limiter la macération, tout en restant en contact avec la plaie tout au long du processus de cicatrisation, afin de garantir une protection de la plaie vis-à-vis de l'environnement extérieur.

La réalisation d'un pansement doit ainsi remplir un cahier des charges complexe et concilier des caractéristiques contradictoires. En particulier, le pansement doit présenter une bonne respirabilité tout en évitant les risques de fuites et de macération, être imperméable aux liquides et aux bactéries tout en étant respirant (c'est-à-dire perméable à la vapeur d'eau), garder sa cohésion quand on le retire, et être facile à fabriquer. Le pansement doit également être facile à poser et rester en place le plus longtemps possible au cours du temps sans altérer la peau périlésionnelle, présenter une capacité d'absorption des exsudats élevée, et ne pas altérer la cicatrisation de la plaie lors de son retrait. Le pansement doit enfin être compatible avec l'utilisation complémentaire d'un système de maintien tel qu'une bande.

Pour favoriser une partie de la gestion des fluides à travers le pansement, il est connu de mettre en œuvre dans ces derniers différentes couches pouvant notamment être constituées de matériaux polymériques et présentant une perméabilité à la vapeur d'eau (MVTR) élevée. On estime en effet qu'au cours d'une journée, la perspiration de la peau génère une perte insensible d'eau d'environ 250 g/m$^2$ [B. Gabard dans l'encyclopédie Médico-Chirurgicale, 50-140-E-10]. Ainsi, pour être considéré comme efficace, un dispositif médical dans son ensemble doit donc présenter une perméabilité à la vapeur d'eau (MVTR) supérieure à cette valeur.

On cherche ainsi toujours à optimiser la perméabilité à la vapeur d'eau de chacune des couches constitutives d'un pansement. En particulier, le développement de la respirabilité de la matrice élastomérique (également appelée masse élastomérique), constituant généralement une couche de contact intermédiaire avec la peau, revêt un intérêt tout particulier. Néanmoins, l'amélioration de la respirabilité de la matrice élastomérique s'accompagne généralement d'une diminution très importante du pouvoir adhésif de ladite matrice sur la peau. En effet, pour favoriser la perméabilité à la vapeur d'eau, il est d'usage d'ajourer la matrice, ce qui limite la surface de contact de cette dernière avec la peau, diminuant ainsi son pouvoir adhésif.

La demande WO 2008074333 de la société Coloplast décrit d'améliorer la perméabilité de masses élastomériques et décrit une matrice adhésive pour dispositif médical comprenant un élastomère tribloc de type S-I-S et un sel hydrosoluble afin d'augmenter la perméabilité à la vapeur d'eau à des niveaux de 20 g/m$^2$/24h.

On demeure néanmoins à la recherche de compositions pour matrices élastomériques entrant dans la constitution de tout dispositif, et notamment de tout dispositif à visée médicale, qui présentent des propriétés améliorées de perméabilité à la vapeur d'eau, tout en conservant un pouvoir adhésif suffisant.

La présente invention propose une composition, notamment susceptible d'être mise en œuvre sous la forme d'une matrice élastomérique permettant de répondre à l'ensemble de ces problématiques, ainsi qu'un dispositif médical la mettant en œuvre.

## RESUME DE L'INVENTION

Ainsi, la présente invention a permis de mettre au point une composition spécifique à base de copolymères à blocs styrèniques, au moins un plastifiant et des particules d'un polymère réticulé ayant une densité de groupement carboxylate comprise entre 2,0 et 12,0 meq/g et une taille de pore moyenne comprise entre 0,005 et 1,0 μm, permettant la préparation d'une matrice élastomérique susceptible d'être mise en œuvre dans un dispositif, de préférence un dispositif médical tel qu'un pansement, ladite matrice présentant une très bonne perméabilité à la vapeur d'eau (MVTR), tout en conservant un pouvoir adhésif suffisant (PA).

Plus précisément, il a été découvert, et ceci constitue le fondement de la présente invention, qu'en associant, dans une composition, au moins un copolymère à blocs styrèniques, un plastifiant, et des particules spécifiques d'un polymère réticulé ayant une densité de groupement carboxylate particulière et une taille de pore elle aussi particulière, il était possible, de façon tout à fait surprenante, d'optimiser la diffusion de la vapeur d'eau au travers de la composition, notamment lorsque celle-ci est mise en œuvre sous forme de matrice élastomérique. L'amélioration des propriétés de perméabilité à la vapeur d'eau de la composition permet, lorsqu'elle est mise en œuvre dans un dispositif médical tel qu'un pansement, d'optimiser le taux d'humidité au niveau de la peau, favorisant ainsi une meilleure cicatrisation. Ainsi selon un premier aspect, la présente invention a pour objet une composition, notamment utile pour la fabrication de pansements, comprenant:

- pour 100 parties en poids, d'au moins un copolymère à blocs styrèniques,
- de 12,5 à 8000 parties en poids, d'au moins un plastifiant,
- de 25 à 4000 parties en poids, de particules d'un polymère réticulé ayant une densité de groupement carboxylate comprise entre 2,0 et 12,0 meq/g et une taille de pore moyenne comprise entre 0,005 et 1,0 μm.

## DESCRIPTION DETAILLEE

Copolymère à blocs styrèniques

La composition selon l'invention, comprend au moins un copolymère à blocs styrèniques, de préférence tribloc de type styrène-oléfine saturée-styrène.

On utilise de préférence des copolymères tribloc constitués d'une association de blocs polystyrène et de blocs polyoléfine de type polystyrène-b-poly (éthylène-butylène)-b-polystyrène (S-EB-S), ou polystyrène-b-poly(éthylène-propylène)-b-polystyrène (S-EP-S), ou polystyrène-b-poly(éthylène-éthylène/propylène)-b-polystyrène (S-EEP-S) de poids moléculaire élevé et ultra-élevé, poly (styrène-isoprène-styrène) (SIS) ou poly (styrène-isobutylène-styrène) (SIBS).

Alternativement, l'un de ces élastomères triblocs peut être remplacé par un élastomère dibloc également constitué d'une association de blocs polystyrène et de blocs polyoléfine, sous réserve que cet élastomère présente des propriétés similaires, en terme de poids moléculaire à l'élastomère tribloc auquel il a été substitué .

De manière avantageuse, on utilise :

- des copolymères triblocs hydrogénés polystyrène-b-poly (éthylène-butylène)-b-polystyrène (S-EB-S, par exemple le Kraton® G 1651 EU, le Kraton G® 1650 E ou le Kraton® G 1654 ES de Kraton Polymers)
- des copolymères triblocs hydrogénés polystyrène-b-poly (éthylène-propylène)-b-polystyrène (S-EP-S, par exemple le Kraton® G 1701 ou G 1702 de Kraton Polymers)
- des copolymères triblocs hydrogénés polystyrène-b-poly (éthylène-éthylène/ propylène)-b-polystyrène (S-EEP-S, tels que les Septon® 4055, 4077 ou 4099 de Kuraray),
- des copolymères triblocs poly (styrène-isoprène-styrène) et en particulier un copolymère tribloc poly (styrène-isoprène-styrène) en mélange avec un copolymère dibloc styrène-isoprène (SIS/SI, tels que les Vector®4114A de TSRC),
- des copolymères triblocs poly (styrène-isobutylène-styrène) (SIBS tels que les Sibstar™ 073T et 103T de Kaneka),

ou un mélange de ceux-ci.

Néanmoins, selon un mode de réalisation alternatif, la composition selon l'invention peut comprendre de préférence un mélange d'au moins deux élastomères tribloc de type S-EB-S ou S-EP-S, quelque soit le poids moléculaire de chacun de ces au moins deux élastomères. Ainsi, par exemple, la composition selon l'invention peut comprendre un élastomère de type Kraton® G 1650 E et un Kraton® G 1654 ES.

Selon un autre mode particulier de réalisation, la composition selon l'invention peut comprendre de préférence un mélange d'au moins deux élastomères tribloc de type S-EB-S ou S-EP-S, en particulier de type S-EEP-S, l'un des deux élastomères présentant un poids moléculaire supérieur ou égal à 200 000 Dalton, de préférence supérieur ou égal à 230 000 Dalton et inférieur ou égal à 350 000 Dalton tel que mesuré par chromatographie par perméation de gel, et l'autre élastomère présentant un poids moléculaire supérieur ou égal à 320 000 Dalton et inférieur ou égal à 450 000 Dalton tel que mesuré par chromatographie par perméation de gel. Selon un mode de réalisation préféré, on choisit un mélange d'au moins deux élastomères tribloc de type S-EB-S ou S-EP-S, en particulier de type S-EEP-S, l'un des deux élastomères présentant un poids moléculaire supérieur ou égal à 240 000 Dalton et inférieur ou égal à 310 000 Dalton tel que mesuré par chromatographie par perméation de gel, et l'autre élastomère présentant un poids moléculaire supérieur ou égal à 320 000 Dalton et inférieur ou égal à 400 000 Dalton tel que mesuré par chromatographie par perméation de gel.

De manière encore plus avantageuse, toujours selon ce mode particulier de réalisation, chaque élastomère possède une viscosité Brookfield d'au moins 5000 mPa.s (en solution à 10 % en poids dans le toluène à 30°C).

Dans le cadre du présent mode de réalisation décrit, le poids moléculaire désigne le poids moléculaire moyen en poids.

On détermine le poids moléculaire moyen en poids de chaque élastomère (Mw) selon la même méthode que celle exposée dans le brevet EP 0 640 115. Ainsi cette détermination se fait par chromatographie par perméation de gel (GPC) dans les conditions suivantes :

Solvant : THF pour HPLC

Vitesse d'écoulement : 1,33 ml/min Température : ambiante

Volume injecté : 200 microlitres

Concentration de l'échantillon : 0,05%

Standard international : phériyl-hexane

Détecteur : Détecteur différentiel d'indice de réfraction Colonnes, aux nombres de deux, de 60 cm de chez Polymer Labs., gel mélangé 10 microns.

Exploitation des données : Logiciel de calibrage de Polymer labs.

On pèse soigneusement les échantillons (100 mg) et on les dissout dans 40 ml de THF dans des ballons de 50 ml. Ensuite, on ajoute 50 microlitres de marqueur quand le polymère est dissous et on complète les solutions jusqu'à la marque de 50 ml. Ensuite, on les filtre à travers un filtre sous pression de 0,2 μM et on les injecte dans la GPC. On prépare 4 solutions séparées de différents mélanges de standards de polystyrène dans des flacons de verre jaugés et on y ajoute un volume connu de marqueur.

On utilise le marqueur pour corriger les variations de flux. Le logiciel crée une courbe d'étalonnage à partir des mélanges de standards en utilisant un ajustement polynomial du troisième ordre.

Les valeurs obtenues pour différents élastomères sont répertoriées dans le tableau suivant :

| Elastomère | Poids moléculaire (Mw) |
|---|---|
| Septon®4055 | 308 000 Dalton |
| Septon®4077 | 392 000 Dalton |
| Septon®4155 | 290 000 Dalton |
| Septon®2005 | 257 000 Dalton |
| Septon®2006 | 251 000 Dalton |
| Septon®2105 | 275 000 Dalton |
| Septon®2055 | 250 000 Dalton |
| Kraton®G 1651 EU | 240 000 Dalton |

Ainsi, une composition comprenant une association d'un élastomère de la marque Kraton® tel que le Kraton®

G 1651 EU ou d'un autre copolymère de la marque Kraton® de la série G possédant la propriété de poids moléculaire souhaitée, à un élastomère de la marque Septon®, tel que le Septon® 4077 ou le Septon® 4099 est envisagée par ce mode particulier de réalisation de l'invention.

De la même manière, une matrice comprenant une association d'un copolymère de la marque Septon® tel que le Septon® 4055 ou d'un autre copolymère de la marque Septon® répertorié dans le tableau précédent ou encore d'un quelconque copolymère de la marque Septon® ayant les propriétés mentionnées précédemment et non répertorié dans le tableau précédent, à un autre copolymère de la marque Septon® tel que le Septon® 4077 ou bien le Septon® 4099 est envisagée par ce mode particulier de réalisation de l'invention.

Selon un mode de réalisation préféré de l'invention, les 100 parties en poids de copolymère à blocs styrèniques représentent 1 à 40% en poids de la composition, de préférence 3 à 30% en poids.

Selon un premier mode de réalisation plus préféré, lorsque le copolymère à blocs styrèniques est constitué d'une association de blocs polystyrène et de blocs polyoléfine de type polystyrène-b-poly (éthylène-butylène)-b-polystyrène (S-EB-S), ou polystyrène-b-poly(éthylène-propylène)-b-polystyrène (S-EP-S), ou polystyrène-b-poly(éthylène-éthylène/propylène)-b-polystyrène (S-EEP-S) de poids moléculaire élevé et ultra-élevé, les 100 parties en poids copolymère à blocs styrèniques représentent 1 à 20 % en poids de la composition, de préférence 3 à 10% en poids.

Selon un second mode de réalisation plus préféré, lorsque le copolymère à blocs styrèniques est constitué d'une association de blocs polystyrène et de blocs polyoléfine de type poly (styrène-isoprène-styrène) (SIS) ou poly (styrène-isobutylène-styrène) (SIBS), les 100 parties en poids copolymère à blocs styrèniques représentent 10 à 40 % en poids de la composition, de préférence 10% à 30% en poids.

Particules de polymère réticulé

Les compositions selon l'invention comprennent des particules d'un polymère réticulé ayant une densité de groupement carboxylate comprise entre 2,0 et 12,0 meq/g et une taille de pore moyenne comprise entre 0,005 et 1,0 $\mu$m.

La « taille moyenne des pores » désigne une taille moyenne exprimée en volume, dont la valeur peut être calculée par la formule 4 V/S où S est la surface spécifique et V est le volume de pores par unité de masse obtenu à partir d'une distribution de taille de pore mesurée par la méthode de compression au mercure.

La taille moyenne des pores, exprimée en volume, peut être déterminée par toute méthode connue de l'homme du métier, par exemple par porosimétrie à intrusion de mercure ou sorptométrie par adsorption d'azote.

Par exemple, la sorptométrie par adsorption d'azote peut être réalisée au moyen d'un appareil TriStar II Micromeritics couplé à un Smart VacPrep Micromeritics. Le lot à caractériser est par exemple soumis à une phase de dégazage pendant 24 heures à température ambiante puis 5 heures à 50°C. La température en cours d'essai est de -196°C, et la pression maintenue dans une gamme de 0 < P/P0 < 0,30 avec P0 = pression de vapeur saturante de l'azote.

La porosimétrie à intrusion de mercure peut être mise en œuvre au moyen d'une cellule de mesure pour poudre ayant un volume de 3cm3 et un capillaire de volume de 0,387cm3. L'analyse est réalisée en deux temps : dans un premier temps l'ensemble « pénétromètre-échantillon » est en configuration « basse pression » (mesure de 0,52 psia (vide primaire) jusqu'à 30 psia soit 2 bars) ; dans un second temps l'ensemble « pénétromètre-échantillon » est en configuration « haute pression » (mesure jusqu'à 60000psia soit 4000 bars). La taille minimale des pores accessibles est de 3 mm.

Selon un mode préféré de réalisation, les particules de polymère réticulé mises en œuvre dans le cadre de la présente demande présentent une surface spécifique inférieure à 1 m$^2$/g. La surface spécifique peut notamment être mesurée par la méthode BET d'adsorption physique, bien connue de l'homme du métier.

Selon un mode préféré de réalisation, le polymère mis en œuvre est un polymère organique.

Le polymère mis en œuvre présente une densité de groupement carboxylate comprise entre 2,0 et 12,0 meq/g. Le groupement carboxylate est un groupe polaire conférant au polymère les propriétés d'absorption d'humidité souhaitées.

Il n'y a pas de limitation particulière quant à la nature du sel mis en œuvre pour la formation des groupements carboxylate. Il peut par exemple s'agir d'un sel de métal alcalin tel que Li, Na, K, Rb et Cs, de métal alcalino-terreux tel que Be, Mg, Mg, Ca, Sr et Ba, d'autres métaux tels que Cu, Zn, Al, Al, Mn, Ag, Fe, Co et Ni, NH4 et des cations organiques tels que des amines.

De préférence, le sel de carboxylate utilisé dans le cadre de la présente invention est le carboxylate de sodium. L'introduction de groupements carboxylates peut être effectuée par toute méthode connue de l'homme du métier. Par exemple, un monomère porteur d'un groupement carboxylate peut être homopolymérisé ou copolymérisé avec d'autres monomères pour donner le polymère selon l'invention. Alternativement, un polymère porteur de groupements carboxylés peut être salifié. Alternativement encore, un polymère peut d'abord être greffé par des groupements carboxylés, lesquels seront ensuite salifiés. Ces méthodes d'introduction de groupements carboxylates sont décrites en détails dans la demande de brevet US 6 080 797.

Un exemple typique de particules de polymère réticulé selon l'invention peut être réalisé à partir d'acrylonitrile ou d'acide méthacrylique.

Plus particulièrement, des microparticules de polymère réticulé, en particulier de polyacrylonitrile, selon l'invention peuvent être obtenues par coagulation, ou par polymérisation par précipitation pour donner un agglomérat de particules de polyacrylonitrile ou un polymère d'acrylonitrile, cet agglomérat ou ce polymère subit une réticulation avec de l'hydrazine ou un dérivé d'hydrazine et enfin une hydrolyse au moins partielle des groupements nitrile résiduels de sorte à obtenir une densité de groupements carboxylate comprise entre 2,0 et 12,0 meq/g. En outre les différentes étapes de ce procédé permettent d'obtenir une taille de pore moyenne comprise entre 0,005 et 1,0 $\mu$m.

A titre illustratif, une première méthode permettant la fabrication d'un polymère réticulé selon l'invention consiste à préparer une solution de polymère à partir d'un polymère acrylonitrile et d'un solvant, à faire ensuite coaguler ladite solution dans un solvant (qui n'est pas un solvant pour ledit polymère acrylonitrile) pour obtenir un polymère acrylonitrile poreux, puis faire réticuler ledit polyacrylonitrile poreux avec une hydrazine, ladite réticulation étant suivie finalement d'une hydrolyse des groupements nitriles résiduels de manière à obtenir une densité de groupement carboxylate comprise entre 2,0 et 12,0 meq/g et une taille de pore moyenne comprise entre 0,005 et 1,0 $\mu$m.

Alternativement, une seconde méthode permettant la fabrication d'un polymère réticulé selon l'invention consiste à faire polymériser par précipitation un mélange de monomères contenant au moins 50% en poids d'acrylonitrile pour obtenir un polymère acrylonitrile poreux, puis réticulation dudit polyacrylonitrile poreux avec une hydrazine et hydrolyse des groupements nitriles résiduels de manière à obtenir une densité de groupement carboxylate comprise entre 2,0 et 12,0 meq/g et une taille de pore moyenne comprise entre 0,005 et 1,0 $\mu$m.

Ces procédés sont décrit plus précisément dans la demande de brevet US 6 080 797.

Néanmoins, toutes particules ayant les propriétés de densité de groupement carboxylate ou de taille de pore requises conviennent à la réalisation d'une composition selon l'invention. Une variante typique consacrerait par exemple la réalisation de telles particules à partir d'acide méthacrylique telles que décrites à l'exemple 5 du brevet US 6 080 797.

Il n'y a pas de limitation particulière quant à la forme des particules de polymère mises en œuvre selon l'invention.

Au sens de la présente invention, le polymère réticulé selon l'invention est caractérisé par une humidité relative d'équilibre (mesurée à 20°C sous une atmosphère à 65% d'humidité relative) comprise entre 20 et 80%, de préférence entre 30 et 70%.

Selon un mode particulier de réalisation, les particules de polymère réticulé selon l'invention présentent une taille moyenne comprise entre 0,1 et 100$\mu$m, de préférence entre 0,3 et 64$\mu$m.

Selon un autre mode particulier de réalisation, les particules d'un polymère réticulé selon l'invention présentent une masse volumique apparente comprise entre 0,1 et 1 g/cm$^3$, de préférence entre 0,2 et 0,7 g/cm$^3$.

De telles particules sont par exemple commercialisées par la société Japan Exlan Co., Ltd sous la dénomination de Taftic® HU 720SF ou Taftic® HU 1200P.

Elles peuvent être introduites dans les compositions selon l'invention sous forme de poudre, ou sous forme de particules en suspension ou en dispersion dans l'eau.

La composition selon l'invention comprend entre 25 et 4000 parties en poids, pour 100 parties en poids d'au moins un copolymère à blocs styréniques, d'un polymère réticulé ayant une densité de groupement carboxylate comprise entre 2,0 et 12,0 meq/g et une taille de pore moyenne comprise entre 0,005 et 1,0 $\mu$m, de préférence de 30 à 3000 parties en poids, de préférence encore de 35 à 2000 parties en poids.

Le plastifiant

La composition selon l'invention comprend au moins un (ou plusieurs) composé(s) plastifiant(s).

Les plastifiants susceptibles d'être utilisés sont bien connus et destinés à améliorer les propriétés d'étirement, de souplesse, d'extrudabilité ou de mise en œuvre des copolymères. On pourra utiliser à cet effet un ou plusieurs plastifiants si nécessaire.

D'une façon générale, en tant que plastifiants, on préférera des composés liquides, compatibles avec la séquence centrale oléfine saturée des copolymères séquencés précités.

Parmi les composés plastifiants susceptibles d'être utilisés à cet effet, on citera en particulier les huiles minérales plastifiantes.

Alternativement, on peut aussi utiliser des produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée.

Dans le cadre de la présente invention, on utilisera de préférence des huiles plastifiantes et en particulier des huiles minérales formées de composés de nature paraffinique ou naphténique, ou de leurs mélanges, dans des proportions variables.

Des huiles minérales plastifiantes particulièrement préférées sont formées de mélanges de composés de nature paraffinique et naphténique.

Parmi les huiles plastifiantes convenant particulièrement, on peut citer les produits commercialisés par la société SHELL sous les dénominations ONDINA® et en particulier ONDINA® 919 ou 933, ou l'huile commercialisée par la société PETRO CANADA sous la référence PURETOL® 9D ou les huiles BLANDOL ou Kaydol commercialisée par Sonneborn ou bien encore les huiles Pionier 2076P ou 2071N commercialisées par Hansen & Rosenthal.

On peut également citer les produits commercialisés par Croda sous la dénomination Crodamol DOA ou commercialisés par Eigenmann & Veronelli sous la dénomination commerciale Lincol DOA-C.

Outre des huiles, le plastifiant peut comprendre de la vaseline. La vaseline mise en œuvre dans les compositions de l'invention est une vaseline conforme à la Pharmacopée Française disponible commercialement.

Dans le cadre de la présente invention, la vaseline est présente en une quantité de 2,5 à 3000 parties en poids, de préférence 12,5 à 2500 parties en poids, pour 100 parties en poids d' d'au moins un copolymère à blocs styrèniques.

Selon une variante de la présente invention, on pourra aussi utiliser une huile minérale associée à une faible quantité d'huile végétale.

Dans le cadre de la présente invention, le plastifiant est présent en une quantité de 12,5 à 8000 parties en poids, de préférence de 375 à 6500 parties en poids pour 100 parties en poids d'au moins un copolymère à blocs styrèniques.

Les résines tackifiantes

La composition selon l'invention peut également comprendre au moins une résine tackifiante.

Parmi les résines tackifiantes susceptibles d'être utilisées selon l'invention, on peut mentionner les résines polyterpènes ou terpènes modifiées, les résines de colophane, les résines hydrocarbonnées, les mélanges de résines cycliques, aromatiques et aliphatiques, ou des mélanges de ces résines.

De tels produits sont commercialisés par exemple :

- par la société ARAKAWA Chemical Industries sous la dénomination ARKON®P qui sont des résines polycyclopentadiènes hydrogénées ;
- par la société EXXON Chemical sous la dénomination ESCOREZ et en particulier la série des résines 5000 qui sont hydrogénées ;
- par la société CRAY VALLEY sous la dénomination WINGTACK®, et en particulier WINGTACK®86 qui est une résine de synthèse formée de copolymères en C5/C9 ou WINGTACK 10 qui est une résine à base de polyterpène synthétique ;
- par la société EASTMAN sous la dénomination KRISTALEX® et en particulier KRISTALEX®3105SD et F100, ou la société ARIZONA CHEMICAL sous la dénomination Sylvares SA100 qui est une résine à base d'alpha-méthylstyrène
- par la société Kolon Industries sous la dénomination Sukorez® de grades SU-90; SU-100; SU- 100S,

De façon générale, afin d'éviter les problèmes de coloration et de stabilité des résines insaturées, on préférera l'utilisation de résines hydrogénées, en particulier avec les copolymères triblocs à séquence centrale saturée car elles sont beaucoup plus compatibles avec ces derniers que les résines insaturées type WINGTACK que l'on utilise essentiellement avec les copolymères triblocs à séquence centrale insaturée.

Les résines tackifiantes peuvent être utilisées seules ou en mélange avec d'autres produits tackifiants, de préférence dans une proportion de 50 à 7000 parties en poids pour 100 parties en poids d'au moins un copolymère à blocs styrèniques et plus particulièrement de 75 à 6000 parties en poids.

Les hydrocolloïdes et autres particules absorbantes

Selon un mode de réalisation de l'invention, les compositions selon l'invention comprennent des particules hydrophiles d'un hydrocolloïde (ou particules d'hydrocolloïde).

Ces particules permettent en effet le retrait indolore d'un pansement interface et le maintien d'un milieu humide au niveau de la plaie afin de favoriser la cicatrisation.

A cet effet, une quantité faible de particules hydrophiles d'un hydrocolloïde est ainsi soit disposée en surface de la matrice élastomérique une fois celle-ci formée soit, de préférence, dispersée de façon homogène au sein de la composition selon l'invention.

Par « hydrocolloïde » ou « particules d'hydrocolloïde », on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium.

Enfin, en alternative aux hydrocolloides, des particules absorbantes peuvent être utilisées. Parmi celles-ci on trouve, des polymères synthétiques à base de sels de l'acide acrylique, connus sous l'appellation "superabsorbants", ou encore les produits commercialisés par la société CIBA Specialty Chemicals sous la dénomination SALCARE® SC91. Une autre référence d'intérêt est celle commercialisée sous la dénomination Aquakeep par Sumitomo, de préférence la référence Aquakeep 10SH-NF.

Les hydrocolloïdes préférés dans le cadre de la présente invention sont les sels de métal alcalin de la carboxyméthylcellulose, et en particulier la carboxyméthylcellulose de sodium (CMC) comme le produit commercialisé BLANOSE 7H4XF PH par la société ASHLAND.

La taille des particules d'hydrocolloïde est généralement comprise entre 50 et 100 microns, avantageusement de l'ordre de 80 microns.

D'une façon générale, la quantité de particules d'hydrocolloïde incorporées dans la composition selon l'invention sera avantageusement de l'ordre de 5 à 2000parties en poids, de préférence de 12.5 à 1800 parties en poids, et de préférence encore de 25 à 1500 parties en poids, pour 100 parties en poids d'au moins un copolymère à blocs styréniques.

Les antioxydants

La composition selon l'invention peut également comprendre des agents antioxydants.

Par « agents antioxydants », on entend désigner ici les composés couramment employés par l'homme de l'art pour assurer la stabilité des composés entrant dans la formulation des compositions, en particulier vis-à-vis de l'oxygène, la chaleur, l'ozone ou les rayonnements ultra-violets.

Comme exemples d'agents antioxydants appropriés, on peut citer notamment les antioxydants phénoliques comme en particulier les produits commercialisés par la société BASF sous les dénominations IRGANOX® 1010, IRGANOX® 565, IRGANOX® 1076 ou les antioxydants soufrés comme par exemple le dibutyldithiocarbamate de zinc commercialisé par la société AKZO sous la dénomination PERKACIT®ZDBC.

Dans le cadre de la présente invention, on préférera l'association de l'IRGANOX 1010 et du PERKACIT®ZDBC.

D'une façon générale ces agents antioxydants pourront être utilisés seuls ou en association en une quantité de l'ordre de 0,125 à 200 parties en poids, de préférence de 0,250 à 100 parties en poids, pour 100 parties en poids d'au moins un copolymère à blocs styréniques.

Dans le cadre de la présente invention, on préférera l'utilisation du produit IRGANOX® 1010 en une quantité comprise entre 0,125 et 100 parties en poids, pour 100 parties en poids d'au moins un copolymère à blocs styréniques.

Actifs additionnels

Outre les agents antioxydants, la composition selon l'invention peut comprendre une (ou plusieurs) autre(s) substance(s) active(s) permettant d'induire ou d'accélérer la cicatrisation ou pouvant avoir un rôle favorable dans le traitement cutané.

Parmi ces substances actives, on peut citer, en particulier, à titre d'exemples:

- les agents favorisant la cicatrisation tels que le rétinol, la vitamine A, la vitamine E, la N-Acétyl Hydroxyproline, les extraits de Centella Asiatica, la papaïne, les huiles essentielles de thym, niaouli, romarin, sauge, l'acide hyaluronique, le sucrose octasulfate de potassium, le sucralfate, l'allantoïne, la metformine
- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique;

Ces agents actifs pourront être utilisés en une quantité de l'ordre de 0,025 à 2000 parties en poids, de préférence de 2,5 à 1500 parties en poids, et de préférence encore de 5 à 1000 parties en poids, pour 100 parties en poids d'au moins un copolymère à blocs styréniques.

Bien entendu, la composition selon l'invention peut aussi comprendre un ou plusieurs autres composés connus pour leur action dans la phase de détersion comme par exemple :

- des enzymes ;
- l'urée.

Adjuvants

A titre d'adjuvants susceptibles d'être utilisés dans les compositions selon l'invention, on peut citer des composés connus pour favoriser le relargage des agents actifs, comme par exemple les produits Montanox® 80 PHA Premium ou Sepinov® EMT 10 ou Sepineo DERM qui sont couramment utilisés dans les produits URGOTUL® qui incorporent des agents actifs.

Ces adjuvants pourront être utilisés en une quantité de l'ordre de 2,5 à 1500 parties en poids, pour 100 parties en poids d'au moins un copolymère à blocs styréniques.

Bien évidemment les modes de réalisation particuliers qui viennent d'être décrits peuvent être mis en œuvre séparément ou selon l'une quelconque de leurs combinaisons.

Matrice élastomérique

Afin de réaliser tout dispositif, et de préférence un pansement, les compositions selon l'invention peuvent être mises en forme à chaud par coulage, trempage, moulage ou encore transfert, pour former une matrice élastomérique.

L'invention a également pour objet, selon un autre aspect, une matrice élastomérique obtenue à partir d'une composition selon l'invention telle que décrite précédemment.

Cette matrice peut être continue, c'est-à-dire pleine et ne présentant pas de perforation, ou discontinue, c'est-à-dire présentant au moins une perforation ou des trous traversants. De préférence, ladite matrice élastomérique est continue.

Les trous traversants peuvent être réalisés par perforation ou poinçonnage d'une composition selon l'invention préalablement formée en couche mince, seule ou associée à un support provisoire.

Alternativement, les matrices élastomériques conformes à l'invention peuvent être fabriquées par coulage à chaud d'une composition telle que décrite précédemment sur un support gravé (plaque ou cylindre) avec le motif retenu pour former des trous traversants, suivi d'un refroidissement et d'un démoulage.

Les trous traversants peuvent être de géométrie quelconque et présenteront par exemple une section transversale circulaire, rectangulaire, trapézoïdale ou carrée.

Leur surface sera généralement comprise entre 1 et 7 mm$^2$.

Ces trous seront répartis, de préférence de façon régulière, avec une densité telle que la surface totale des trous représente entre 20 et 70 %, et de préférence entre 30 et 60 % de la surface totale du pansement.

D'une façon générale, les matrices élastomériques conformes à l'invention présenteront une épaisseur comprise entre 0,1 $\mu$m et 5 mm, et de préférence entre 20 $\mu$m et 2 mm.

Il peut également être envisagé d'utiliser cette matrice élastomérique pour enduire une armature ou un support.

Dispositif

L'invention a ainsi pour objet, selon un mode préféré de réalisation, un dispositif médical tel qu'un patch, un film, une bande ou encore un pansement, ou bien un dispositif de type textile fonctionnel tel qu'un article de sport. De préférence, ledit dispositif est un pansement caractérisé en ce qu'il comprend une matrice élastomérique telle que précédemment décrite.

Selon un mode de réalisation préféré, la présente demande vise à couvrir un pansement comprenant une matrice élastomérique se présentant sous la forme d'une couche mince obtenue à partir d'une composition comprenant :

- pour 100 parties en poids d'au moins un copolymère à blocs styrèniques
- de 12,5 à 8000 parties en poids, d'au moins un plastifiant,
- de 25 à 4000 parties en poids, de particules d'un polymère réticulé ayant une densité de groupement carboxylate comprise entre 2,0 et 12,0 meq/g et une taille de pore moyenne comprise entre 0,005 et 1,0 $\mu$m.

La matrice élastomérique peut être utilisée seule pour constituer le dispositif ou en association avec une ou plusieurs couches, telles que des mousses, des textiles, des matériaux composites ou encore des films.

La matrice élastomérique peut se trouver à n'importe quel niveau du dispositif, c'est-à-dire constituer l'une quelconque des différentes couches du dispositif, et peut être en contact avec la peau, la plaie.

La présente invention est illustrée dans les exemples non limitatifs présentés ci-après.

## EXEMPLES

### Exemple 1

Préparation des compositions

Les compositions des exemples 1 à 14 ont été élaborées à l'aide des constituants suivants dans les proportions, exprimées en parties en poids, mentionnées dans le tableau 1 ci-dessous.

Elastomère : comprend au moins un copolymère séquencé de poly (styrène-oléfine -styrène) (en abrégé par exemple SEBS, SEEPS, SIS/SI ou SIBS respectivement) :

- KRATON G 1654 ES
- KRATON G 1650 E
- Kraton G 1651 EU
- Septon 4055
- Vector 4114A
- Sibstar 103T

Plastifiant : huile minérale Ondina® 919 ou 933 commercialisées par la société SHELL, Pionier 2076P ou 2071N commercialisées par Hansen & Rosenthal ou Blandol commercialisée par Sonneborn.

Vaseline : vaseline Codex® A commercialisée par la société AIGLON

Antioxydant : IRGANOX® 1010 commercialisé par la société BASF et/ou PERKACIT®ZDBC par la société AKZO.

Hydrocolloïde : Carboxyméthylcellulose sodique CMC BLANOSE® 7H4XFPH commercialisée par la société ASHLAND, ou particules absorbantes Aquakeep 10SH-NF de Sumitomo

Résines :

- Escorez 5380, résine hydrocarbonée cycloaliphatique, présentant un point de ramollissement situé entre 80-90°C, commercialisée par Exxon Mobil,
- Sukorez SU-100S, résine polycyclopentadiene présentant un point de ramollissement situé entre 97 et 106°C, commercialisée par Kolon Industries

Particules de polymère réticulé:

- Taftic HU-720 SF de la société Japan Exlan Co., Ltd
- Taftic HU-1200 P de la société Japan Exlan Co., Ltd

Fabrication de la composition

Dans un dispositif de mélange à chaud, on a introduit dans un premier temps le plastifiant et/ou la vaseline à une température inférieure à 90°C, puis dans une seconde étape, le(s) copolymère(s) et l'antioxydant sont ajoutés au mélange précédent à une température de consigne comprise entre 160 et 180°C. Dans une dernière étape, sont ajoutés les autres composés parmi lesquels l'hydrocolloïde ou la particule absorbante, la résine, ainsi que les particules de polymère réticulé selon l'invention jusqu'à l'obtention d'un mélange homogène.

Enfin, on vidange le dispositif de mélange à chaud.

Mesure de la perméabilité à la vapeur d'eau :

Les conditions de réalisation du test se basent sur la norme NF EN 13726 - liquides en contact.

MATERIEL / APPAREILLAGE

Balance résolution 0,1 mg
Emporte-pièce diamètre 44mm,
Cellule de mesure en aluminium de diamètre D = 35,7 mm et surface 10 cm$^2$
Fiole jaugée (= 25mL) ou pompe doseuse
Etuve thermostatée à 37°C et < 20% d'humidité relative (HR) à brassage d'air
Pièce climatisée à 21°C +/-2 - HR à 60% +/- 15
Réactifs : eau déminéralisée et solution NaCl, CaCl$_2$.

ECHANTILLONNAGE/ CONDITIONNEMENT DES ECHANTILLONS

Nombre d'éprouvette n ≥ 5.
Température T = 37°C ± 2 °C.
Hygrométrie HR < 20%

MODE OPERATOIRE

Les conditions de réalisation du test se basent sur la norme NF EN 13726 - liquides en contact, étaient les suivantes.

Le principe de cette mesure est le suivant :

1. Verser à l'intérieur de la cellule un volume V de liquide
2. Positionner, sur l'ouverture de la cellule de mesure, l'échantillon à tester (face adhésive sur le joint siliconé si le produit est adhésivé)
3. Positionner par-dessus l'échantillon, centré pour éviter les fuites, le dispositif de maintien puis visser les 3 vis jusqu'en butée
4. Peser l'ensemble → $P_{MVTR0}$
5. Retourner les cellules pour mettre le liquide en contact avec l'échantillon. Placer cet ensemble dans une étuve à une température T pendant un temps t (t exprimé en heure)
6. Peser dans les 5 minutes suivant la fin du test (t) l'ensemble du dispositif → $P_{MVTR1}$

EXPRESSION DES RESULTATS

Calculer le taux de transmission à la vapeur d'eau (MVTR)

$$MVTR1 = (P_{MVTR0} - P_{MVTR1})/(\pi D^2/4)$$

$$MVTR1 = 4 (P_{MVTR0} - P_{MVTR1})/\pi D^2$$

Soit, dans les conditions standard, MVTR1 = $(P_{MVTR0} - P_{MVTR1})/(10*10^{-4})$

Exprimer le résultat en g/m$^2$/24h.

Calculer la moyenne des n essais, au g/m$^2$ près.

$$MVTR = \frac{1}{n}\sum MVTR_i$$

Les quantités de chacun des composés introduits dans chacune des compositions 1 à 14 sont données en parties en poids dans le tableau 1.

Tableau 1

| | Compo 1 | Compo 2 | Compo 3 | Compo 4 | Compo 5 | Compo 6 | Compo 7 | Compo 8 | Compo 9 | Compo 10 | Compo 11 | Compo 12 | Compo 13 | Compo 14 |
|---|---|---|---|---|---|---|---|---|---|---|---|---|---|---|
| Pionier 2076P | 741 | 736 | 741 | 736 | | | | | 741 | 732 | | | 780 | 774 |
| Pionier 2071N | | | | | 70 | 70 | | | | | | 70 | | |
| Blandol | | | | | | | 70 | 70 | | | | | | |
| Ondina 933 | | | | | | | | | | | 70 | | 100 | 100 |
| Vaseline Codex A | | | | | | | | | 180 | 179 | | | | |
| Kraton G 1651 EU | 100 | 100 | | | | | | | | | | | | |
| Septon 4055 | | | 100 | 100 | | | | | | | | | | |
| Vector 4114A | | | | | 100 | 100 | | | | | 100 | 100 | | |
| Sibstar 103T | | | | | | | 100 | 100 | | | | | | |
| Kraton G 1650 E | | | | | | | | | 32 | 32 | | | | |
| Kraton G 1654 ES | | | | | | | | | 68 | 68 | | | | |
| Irganox 1010 | 4 | 5 | 4 | 5 | 1,5 | 2 | 1,5 | 2 | 2,5 | 3 | 2 | 2 | 3 | 4 |
| Perkacit ZDBC | | | | | 1,5 | 2 | 1,5 | 2 | | | 2 | 2 | | |
| Escorez 5380 | 1115 | 1109 | 1115 | 1109 | 150 | 150 | 150 | 150 | | | 150 | 150 | | |
| Sukorez SU-100S | | | | | | | | | | | | | 729 | 722 |

| | Compo 1 | Compo 2 | Compo 3 | Compo 4 | Compo 5 | Compo 6 | Compo 7 | Compo 8 | Compo 9 | Compo 10 | Compo 11 | Compo 12 | Compo 13 | Compo 14 |
|---|---|---|---|---|---|---|---|---|---|---|---|---|---|---|
| Taftic HU-1200 P | | | | | | | | | | | | | | 400 |
| Taftic HU-720 SF | | 488 | | 488 | | 80 | | 80 | | 298 | | 120 | | |
| CMC Blanose 7H4XFPH | | | | | | | | | 180 | 179 | | | | |
| Aquakeep 10SH-NF | | | | | | | | | | | 120 | | | |

Résultats :

Tableau 2

| | Perméabilité à la vapeur d'eau (MVTR à 37°C en g/m$^2$/24h) | Variation de la perméabilité à la vapeur d'eau |
|---|---|---|
| Composition 1 | 51 | +35 588% |
| Composition 2 | 18201 | |
| Composition 3 | 48 | +38 242% |
| Composition 4 | 18404 | |
| Composition 5 | 55 | +15 867 % |
| Composition 6 | 8782 | |
| Composition 7 | 24 | + 2 817% |
| Composition 8 | 700 | |
| Composition 9 | 91 | + 4 085% |
| Composition 10 | 3808 | |
| Composition 11 | 26 | + 62 950 % |
| Composition 12 | 16367 | |
| Composition 13 | 81 | + 2770 % |
| Composition 14 | 2244 | |

L'ajout de particules selon l'invention constituées d'un polymère réticulé ayant une densité de groupement carboxylate comprise entre 2,0 et 12,0 meq/g et une taille de pore moyenne comprise entre 0,005 et 1,0 μm permet d'augmenter de manière drastique la perméabilité à la vapeur d'eau (MVTR) des compositions.

**Exemple 2**

Préparation des compositions

Les compositions suivantes 15 à 19 ont été élaborées à l'aide des constituants suivants dans les proportions, exprimées en parties en poids, mentionnées dans le tableau 3 ci-dessous.

Elastomère : copolymère séquencé de poly (styrène-éthylène-butylène-styrène) (en abrégé SEBS) :

- Kraton G 1651 EU

Plastifiant : huile minérale Pionier 2076P commercialisée par Hansen & Rosenthal ou Blandol commercialisée par Sonneborn.
Antioxydant : IRGANOX 1010 par la société BASF.

Résine :

- Escorez 5380, résine hydrocarbonée cycloaliphatique, présentant un point de ramollissement situé entre 80-90°C, commercialisée par Exxon Mobil, ou,
- Sukorez SU-100S, résine polycyclopentadiene présentant un point de ramollissement situé entre 97 et 106°C, commercialisée par Kolon Industries

Particules de polymère réticulé:

- Taftic HU-720 SF la société Japan Exlan Co., Ltd

Fabrication de la composition

Dans un dispositif de mélange à chaud, on a introduit dans un premier temps le plastifiant et/ou la vaseline à une température inférieure à 90°C, puis dans une seconde étape, le(s) copolymère(s) et l'antioxydant sont ajoutés au mélange précédent à une température de consigne de 160°C. Enfin, dans une dernière étape, sont ajoutés les autres composés parmi lesquels l'hydrocolloïde ou la particule absorbante, la résine, ainsi que les particules de polymère réticulé selon l'invention jusqu'à l'obtention d'un mélange homogène. Enfin, on vidange le dispositif de mélange à chaud.

Mesure de la perméabilité à la vapeur d'eau :

Les conditions de réalisation du test se basent sur la norme NF EN 13726 - liquides en contact.

MATERIEL / APPAREILLAGE

Balance résolution 0,1 mg
Emporte-pièce diamètre 44mm,
Cellule de mesure en aluminium de diamètre D = 35,7 mm et surface 10 cm$^2$
Fiole jaugée (= 25mL) ou pompe doseuse
Etuve thermostatée à 37°C et < 20% d'humidité relative (HR) à brassage d'air
Pièce climatisée à 21°C +/-2 - HR à 60% +/- 15
Réactifs : eau déminéralisée et solution NaCl, CaCl$_2$.

ECHANTILLONNAGE / CONDITIONNEMENT DES ECHANTILLONS

Nombre d'éprouvette n $\geq$ 5.
Température T = 37°C $\pm$ 2 °C.
Hygrométrie HR < 20%

MODE OPERATOIRE

Les conditions de réalisation du test se basent sur la norme NF EN 13726 - liquides en contact, étaient les suivantes.
Le principe de cette mesure est le suivant :

7. Verser à l'intérieur de la cellule un volume V de liquide
8. Positionner, sur l'ouverture de la cellule de mesure, l'échantillon à tester (face adhésive sur le joint siliconé si le produit est adhésivé)
9. Positionner par-dessus l'échantillon, centré pour éviter les fuites, le dispositif de maintien puis visser les 3 vis jusqu'en butée
10. Peser l'ensemble → $P_{MVTR0}$
11. Retourner les cellules pour mettre le liquide en contact avec l'échantillon. Placer cet ensemble dans une étuve à une température T pendant un temps t (t exprimé en heure)
12. Peser dans les 5 minutes suivant la fin du test (t) l'ensemble du dispositif → $P_{MVTR1}$

EXPRESSION DES RESULTATS

Calculer le taux de transmission à la vapeur d'eau (MVTR)

$$MVTR1 = (P_{MVTR0} - P_{MVTR1})/(\pi D^2/4)$$

$$MVTR1 = 4\,(P_{MVTR0} - P_{MVTR1})/\pi D^2$$

Soit, dans les conditions standard, MVTR1 = $(P_{MVTR0} - P_{MVTR1})/(10*10^{-4})$

Exprimer le résultat en $g/m^2/24h$.

Calculer la moyenne des n essais, au $g/m^2$ près.

$$MVTR = \frac{1}{n} \sum MVTR_i$$

Les quantités de chacun des composés introduits dans les compositions 15 à 19 sont données en partie en poids dans le tableau 3.

Tableau 3 :

|  | Compo. 15 | Compo. 16 | Compo. 17 | Compo. 18 | Compo. 19 |
|---|---|---|---|---|---|
| Pionier 2076P | 739 | 739 | 739 | 738 | 741 |
| Kraton G1651 EU | 100 | 100 | 100 | 100 | 100 |
| Irganox 1010 | 4,5 | 4,5 | 4.5 | 4,5 | 4 |
| Escorez 5380 | 1113 |  | 1114 | 1112 | 1115 |
| Taftic HU-720 SF | 279 | 279 | 345 | 414 |  |
| Sukorez SU-100S |  | 1113 |  |  |  |

Mesure du pouvoir adhésif à 180° :

La première étape consiste à apposer l'une des compositions 15 à 19 de l'exemple 2 sur un support de type non tissé et à découper ensuite une éprouvette c'est-à-dire une bande de l'une de ces associations de largeur 20 mm et de longueur 300 mm.

En parallèle, dans une deuxième étape, est découpée une bande de papier cartonné de largeur et de longueur supérieures à celles de l'éprouvette.

On applique ensuite l'éprouvette, avec une légère pression du doigt sur le papier cartonné parallèlement à sa plus grande dimension, sans étirer la bande et en évitant d'inclure la moindre bulle d'air.

On fait deux allers retours à l'aide d'un rouleau applicateur, à une vitesse V1 = 10 mm/s et un poids P = 2 kg/cm, sans pression additionnelle, pour obtenir un contact intime entre la masse adhésive et la surface du papier.

On laisse l'ensemble climatiser à une température de 23+/-2°C pendant un temps t = 10 min.

Enfin, dans une étape terminale, on enregistre la force moyenne F du pelage à 180° réalisé à une vitesse V2 = 300 mm/min de l'éprouvette sur le papier cartonné au moyen d'un dynamomètre électronique.

Tableau 4

|  | Perméabilité à la vapeur d'eau (MVTR à 37°C en $g/m^2/24h$) | Pouvoir adhésif à 180° sur papier cartonné (cN/cm) |
|---|---|---|
| Composition 15 | 295 +/- 40 | 88 +/- 8 |
| Composition 16 | 290 +/- 37 | 118 +/- 7 |

(suite)

|  | Perméabilité à la vapeur d'eau (MVTR à 37°C en g/m$^2$/24h) | Pouvoir adhésif à 180° sur papier cartonné (cN/cm) |
|---|---|---|
| Composition 17 | 2102 +/- 431 | 94 +/- 2 |
| Composition 18 | 3048 +/- 60 | 88 +/-7 |
| Composition 19 | 51 +/- 1 | 97 +/- 9 |

L'ajout de particules d'un polymère réticulé ayant une densité de groupement carboxylate comprise entre 2,0 et 12,0 meq/g et une taille de pore moyenne comprise entre 0.005 et 1.0 μm permet d'augmenter de manière drastique la perméabilité à la vapeur d'eau desdites compositions. On remarque en outre un effet-dose de l'introduction de ces particules dans chacune des compositions de l'exemple 2, augmentant ainsi la perméabilité à la vapeur d'eau sans détériorer le pouvoir adhésif desdites compositions.

**Revendications**

1. Composition comprenant:

    - pour 100 parties en poids d'au moins un copolymère à blocs styrèniques,
    - de 12,5 à 8000 parties en poids d'au moins un plastifiant,
    - de 25 à 4000 parties en poids de particules d'un polymère réticulé ayant une densité de groupement carboxylate comprise entre 2,0 et 12,0 meq/g et une taille de pore moyenne comprise entre 0,005 et 1,0 μm.

2. Composition selon la revendication 1, **caractérisée en ce que** le copolymère à blocs styrèniques est choisi parmi

    - un copolymère tribloc styrène-oléfine saturée-styrène, de préférence de type SEPS, SEBS, SEEPS,
    - un copolymère tribloc de type SIS, SIBS

    ou leurs mélanges.

3. Composition selon l'une des revendications 1 ou 2, **caractérisée en ce que** les particules de polymère réticulé présentent une taille moyenne comprise entre 0,1 et 100μm, de préférence entre 0,3 et 64μm.

4. Composition selon l'une des revendications précédentes, **caractérisée en ce que** le polymère réticulé est préparé à partir d'acrylonitrile ou d'acide méthacrylique.

5. Composition selon l'une des revendications précédentes, **caractérisée en ce que** le plastifiant est constitué d'une huile minérale, de vaseline et/ou d'un mélange de ces composés.

6. Composition selon l'une des revendications précédentes, **caractérisée en ce qu'**elle comprend une résine tackifiante, de préférence choisie parmi les résines polyterpènes ou terpènes modifiées, les résines de colophane, les résines hydrocarbonées, les mélanges de résines cycliques, aromatiques et aliphatiques, ou des mélanges de ces résines.

7. Composition selon l'une des revendications précédentes, **caractérisée en ce qu'**elle comprend des particules d'hydrocolloïde et/ou des particules absorbantes.

8. Composition selon l'une des revendications précédentes, **caractérisée en ce qu'**elle comprend une ou plusieurs substance(s) active(s) permettant d'induire ou d'accélérer la cicatrisation ou pouvant avoir un rôle favorable dans le traitement cutané.

9. Matrice élastomérique, **caractérisée en ce qu'**elle est obtenue à partir d'une composition selon l'une des revendications 1 à 8, de préférence par coulage à chaud de ladite composition.

10. Matrice élastomérique selon la revendication 9, **caractérisée en ce qu'**elle se présente sous forme continue ou discontinue, de préférence continue.

**11.** Dispositif, **caractérisé en ce qu'**il comprend une matrice élastomérique selon l'une des revendications 9 ou 10.

**12.** Dispositif selon la revendication 11, **caractérisé en ce qu'**il s'agit d'un pansement.

**Patentansprüche**

**1.** Zusammensetzung, welche Folgendes umfasst:

- auf 100 Gewichtsteile wenigstens eines Styrol-Blockcopolymers
- 12,5 bis 8000 Gewichtsteile wenigstens eines Weichmachers,
- 25 bis 4000 Gewichtsteile von Partikeln eines vernetzten Polymers mit einer Carboxylatgruppendichte von 2,0 bis 12,0 meq/g und einer durchschnittlichen Porengröße von 0,005 bis 1,0 $\mu$m.

**2.** Zusammensetzung nach Anspruch 1, **dadurch gekennzeichnet, dass** das Styrol-Blockcopolymer gewählt ist aus

- einem mit Styrol gesättigten Olefin-Styrol-Triblock-Copolymer, bevorzugt vom Typ SEPS, SEBS, SEEPS,
- einem Triblock-Copolymer vom Typ SIS, SIBS
oder Mischungen davon.

**3.** Zusammensetzung nach einem der Ansprüche 1 oder 2, **dadurch gekennzeichnet, dass** die vernetzten Polymerpartikel eine durchschnittliche Größe von 0,1 bis 100 $\mu$m, bevorzugt von 0,3 bis 64 $\mu$m, aufweisen

**4.** Zusammensetzung nach einem der vorhergehenden Ansprüche, **dadurch gekennzeichnet, dass** das vernetzte Polymer aus Acrylnitril oder Methacrylsäure hergestellt ist.

**5.** Zusammensetzung nach einem der vorhergehenden Ansprüche, **dadurch gekennzeichnet, dass** der Weichmacher aus einem Mineralöl, Vaseline und/oder einer Mischung dieser Verbindungen besteht.

**6.** Zusammensetzung nach einem der vorhergehenden Ansprüche, **dadurch gekennzeichnet, dass** sie ein klebrigmachendes Harz enthält, bevorzugt gewählt aus modifizierten Polyterpen- oder Terpenharzen, Kolophoniumharzen, Kohlenwasserstoffharzen, Mischungen von cyclischen, aromatischen und aliphatischen Harzen oder Mischungen dieser Harze.

**7.** Zusammensetzung nach einem der vorhergehenden Ansprüche, **dadurch gekennzeichnet, dass** sie Hydrokolloidpartikel und/oder absorbierende Partikel enthält.

**8.** Zusammensetzung nach einem der vorhergehenden Ansprüche, **dadurch gekennzeichnet, dass** sie einen oder mehrere Wirkstoffe enthält, die die Wundheilung induzieren oder beschleunigen oder die eine günstige Rolle bei der Hautbehandlung spielen können.

**9.** Elastomer-Matrix, **dadurch gekennzeichnet, dass** sie aus einer Zusammensetzung nach einem der Ansprüche 1 bis 8, bevorzugt durch Heißgießen der Zusammensetzung, erhalten wird.

**10.** Elastomer-Matrix nach Anspruch 9, **dadurch gekennzeichnet, dass** sie in kontinuierlicher oder diskontinuierlicher, bevorzugt kontinuierlicher, Form vorliegt.

**11.** Vorrichtung, **dadurch gekennzeichnet, dass** sie eine Elastomer-Matrix nach einem der Ansprüche 9 oder 10 umfasst.

**12.** Vorrichtung nach Anspruch 11, **dadurch gekennzeichnet, dass** es sich um einen Wundverband handelt.

**Claims**

**1.** Composition comprising:

- for 100 parts by weight of at least one styrene-block copolymer,
- from 12.5 to 8,000 parts by weight of at least one plasticiser,
- from 25 to 4,000 parts by weight of particles of a cross-linked polymer having a carboxylate-group density between 2.0 and 12.0meq/g and an average pore size between 0.005 and $1.0\mu$m.

2. Composition according to claim 1, **characterised in that** the styrene-block copolymer is chosen from :

   - a styrene-saturated olefin-styrene triblock copolymer, preferably of the SEPS, SEBS, SEEPS,
   - a triblock copolymer of SIS, SIBS type,

   or their mixtures.

3. Composition according to one of claims 1 or 2, **characterised in that** the particles of cross-linked polymer have an average size between 0.1 and $100\mu$m, preferably between 0.3 and $64\mu$m.

4. Composition according to one of the previous claims, **characterised in that** the cross-linked polymer is prepared from acrylonitrile or methacrylic acid.

5. Composition according to one of the previous claims, **characterised in that** the plasticiser consists of a mineral oil, of Vaseline and/or of a mixture of these compounds.

6. Composition according to one of the previous claims, **characterised in that** it comprises a tackifying resin, preferably chosen from the modified polyterpene or terpene resins, the rosin resins, the hydrocarbon resins, the mixtures of cyclic, aromatic and aliphatic resins, or mixtures of these resins.

7. Composition according to one of the previous claims, **characterised in that** it comprises particles of hydrocolloid and/or absorbent particles.

8. Composition according to one of the previous claims, **characterised in that** it comprises one or more active substance(s) allowing to induce or accelerate healing or capable of playing a favourable role in skin treatment.

9. Elastomer matrix, **characterised in that** it is obtained from a composition according to one of claims 1 to 8, preferably by hot casting of said composition.

10. Elastomer matrix according to claim 9, **characterised in that** it is in continuous or discontinuous form, preferably continuous.

11. Device, **characterised in that** it comprises an elastomer matrix according to one of claims 9 or 10.

12. Device according to claim 11, **characterised in that** it is a dressing.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008074333 A
- EP 0640115 A

- US 6080797 A